# EUROPEAN PATENT APPLICATION

(11) **EP 3 992 714 A1**
(43) Date of publication of application: **04.05.2022**
(21) Application number: 20794838.1
(22) Date of filing: 10.04.2020
(51) Int. Cl.: G03F 7/20, B23K 26/364, B23K 26/359, B23K 26/03, G03F 7/075, B81C 1/00, H01L 21/027

(54) **MICROPATTERNING METHOD, MICROPATTERNING APPARATUS AND MICROPATTERNING CHIP FOR SILICONE-BASED ELASTOMER**

(30) Priority: 26.04.2019 KR 20190049369; 27.03.2020 KR 20200037522
(71) Applicant: Seoul National University R & DB Foundation, Seoul 08826 (KR)
(72) Inventor: KO, Seung Hwan, Seoul 08826 (KR); SHIN, Jae Ho, Incheon 21997 (KR); WON, Phil Lip, Seoul 08826 (KR); JEONG, Seong Min, Seoul 08826 (KR)
(74) Representative: Tribalyte Ideas
(86) International application number: PCT/KR2020/004868
(87) International publication number: WO 2020/218763

(57) **Abstract**

The present invention relates to a method for micropatterning a silicone-based elastomer, the method forming an initiator at one position of a silicone-based elastomer that has high light transmittance and transparency and moving a laser beam so as to induce chain pyrolysis, thereby enabling the formation of a high-quality micropattern even while performing micropatterning in a very short time.

## Description

### SUMMARY

The present invention relates to a method for micro-patterning a silicone-based elastomer, the method forming an initiator at one position of a silicone-based elastomer that has high light transmittance and transparency, and moving a laser beam so as to induce chain pyrolysis, thereby enabling the formation of a high-quality micro pattern even while performing micro-patterning in a very short time.

### Description of Related Art

The present invention relates to a method for micro-patterning of a silicon-based elastomer, and more particularly, to a method capable of forming a precise micro pattern on a transparent silicon-based elastomer by using a laser.

### Background

Silicone-based elastomers have excellent biocompatibility and can be used for cell culture, and in particular, they have the advantage of being transparent so that the proliferation and differentiation of cells can be observed in real time. Accordingly, various studies are being conducted for manufacturing a micro-patterned chip such as a bio-chip or an organ-on-a-chip through micro-patterning on a silicon-based elastomer, for example, polydimethylsiloxane (PDMS).

In general, a photolithography process is used as a method of micro-patterning on a silicon-based elastomer. However, the photolithography process is very complicated and requires expensive equipment, and in particular, there is a problem in that a mask must be manufactured for each pattern to be formed. The photolithography mask is not only expensive to manufacture, but also requires a long manufacturing time.

Compared to the photolithography process, the method using a laser takes a shorter and simpler time for the micro-patterning process. However, since a silicon-based elastomer having high light transmittance and transparency transmits a laser, it is not easy to perform micro-patterning using a laser.

Due to this problem, there are two major methods for micro-patterning a silicon-based elastomer using a conventional laser.

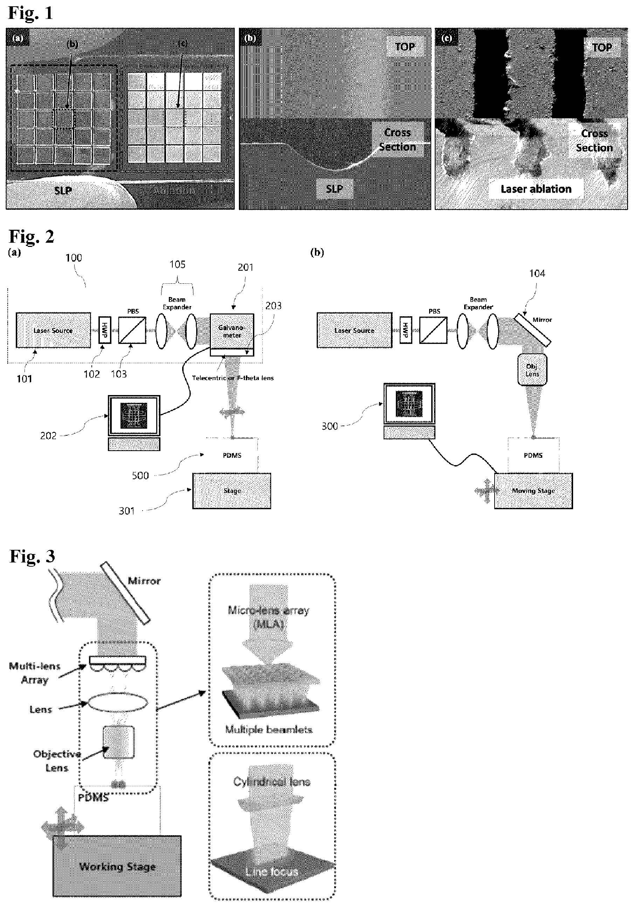

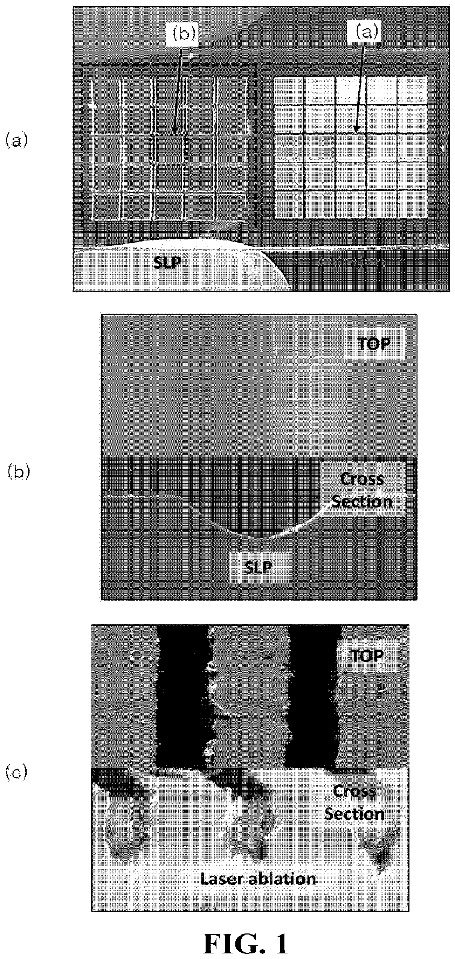

The first method is a method of ablation of the surface of a silicon-based elastomer using a pulsed laser of high energy. However, in this ablation process, the quality of the formed micro pattern is too low, so it is difficult to actually commercialize it. FIG. 1 shows that a micro pattern is formed by using a pulse laser, but problems such as the micro pattern is not uniform and uneven can be confirmed. This is because of the limitations of pulsed lasers using high energy.

The second method is to apply a light-absorbing layer on the surface of a silicon-based elastomer, as shown in Journal of Micromechanics and Microengineering, 26 (2016) 035008 (8pp), Ziya Isiksacan et al., Rapid fabrication of microfluidic PDMS devices from reusable PDMS molds using laser ablation. After formation, micro-patterning is performed using a laser, and the light absorption layer is removed again. However, in the process of forming the light-absorbing layer on the silicone-based elastomer, the surface properties of the silicone-based elastomer are deteriorated due to problems with the adhesive or hydrophobic surface. For example, there is a problem in that transparency is lowered or the surface becomes hydrophobic, making it difficult to use in a microfluid device. In addition, the method of forming the light absorption layer has a very complicated process and is very inefficient in terms of time and cost, making mass production virtually impossible.

Accordingly, the present inventors have developed a new method capable of micro-patterning using a laser without damaging the surface properties of the transparent silicon-based elastomer.

### DETAILED DESCRIPTION OF THE INVENTION

### TECHNICAL CHALLENGE

An object of the present invention is to provide a method for micro-patterning of a silicon-based elastomer having high quality while performing micro-patterning in a very fast time by inducing continuous thermal decomposition using a laser.

Another object of the present invention is to provide a method for micro-patterning of a silicon-based elastomer capable of forming a three-dimensional micro pattern by inducing continuous thermal decomposition using a laser and a laser micro-patterning apparatus.

Another object of the present invention is to provide a microfluidic chip or a cell culture chip having a micro pattern manufactured through continuous thermal decomposition using a laser.

On the other hand, other objects not specified in the present invention will be additionally considered within a range that can be easily inferred from the following detailed description and effects thereof.

### MEANS OF SOLVING THE PROBLEM

In order to achieve the above object, a method for micro-patterning of a silicon-based elastomer according to an example of the present invention comprising: forming an initiator having a first region at one position of the silicone-based elastomer, wherein a laser beam from a micro-patterning device irradiating a laser beam is irradiated to the initiator to induce a first thermal decomposition in the first region, but a second thermal decomposition occurs outside the first region by conduction of heat generated in the first thermal decomposition. to form a second region capable of absorbing light, wherein the laser beam is irradiated while moving to a second region to form a micropattern.

In an example, the silicon-based elastomer has a first surface that is an incident surface to which a laser beam is irradiated, and a second surface opposite to the first surface, and the initiator is formed on the first surface of the silicon-based elastomer to generate a laser beam. 2D micro patterns can be formed while moving within the 2D area.

In one example, the silicon-based elastomer has a first surface, which is an incident surface to which a laser beam is irradiated, and a second surface opposite to the first surface, and the initiator is formed on the second surface of the silicon-based elastomer to generate a laser beam. 3D micro patterns can be formed while moving within the 3D area.

In an example, the micro-patterning device generates a first axis laser beam, a second axis laser beam, and a third axis laser beam, wherein the first axis laser beam, the second axis laser beam, and the third axis laser beam are one Intersecting points are formed at the points, and the 3D micro patterns can be formed while moving in the 3D region using the initiator at one position of the silicon-based elastomer as a starting point.

In one example, the initiator may be a solution or a solid material having a higher light absorption than the silicone-based elastomer. For example, it may be a colored pigment that absorbs light, a dye, an ink, or a solution or a solid containing one or more of these.

In an example, the initiator may be positioned on the surface of the silicone-based elastomer, or may be inserted or embedded therein.

In one example, after forming the micro pattern, the thermal decomposition product may be removed. In this case, the thermal decomposition product may be SiC, SiOC, SiO2 or amorphous silica.

In an example, the silicone-based elastomer may be poly(methylmethacrylate) (PDMS).

In an example, the laser beam may be a continuous wave laser beam or a pulsed laser beam.

In one example, the power density of the laser beam may be a laser beam less than the power density to ablate the silicon-based elastomer.

In order to achieve the above object, a micro-patterning chip of another example of the present invention is manufactured by the above-described micro-patterning method of a silicon-based elastomer, and the formed micro pattern has a turbidity to light of 550 nm of 4%T or more.

In order to achieve the above object, a micro-patterning device of another example of the present invention is composed of a laser beam generating unit, a stage, and a laser beam generating unit or a control unit for controlling the stage, and induces chain thermal decomposition in a silicon-based elastomer to induce a micro pattern. to form In this case, the laser beam generator includes a laser oscillator; mirror unit; beam expander; and a laser beam scanner, mounted on the stage, and a workpiece on which a micro pattern is formed by the laser beam, comprising a silicon-based elastomer including at least one initiator absorbing light, the laser Either the beam scanner or the stage or both are driven into a 2D domain or a 3D domain by a driving device.

In another example, the laser beam generating unit generates a first laser beam generating unit generating a first axis laser beam, a second laser beam generating unit generating a second axis laser beam, and a third axis laser beam and a third laser beam generating unit, wherein the first to third laser beam generating units may be configured to have an intersection point where the first axis laser beam, the second axis laser beam, and the third axis intersect at one point.

In another example, the laser beam scanner may be a galvanometer scanner.

In another example, the wavelength of the laser beam generated by the laser beam generator may be 200 nm to 1,000 nm.

In another example, the energy density of the laser beam generated by the laser beam generator may be 10 to 100 J/m.

### EFFECTS OF THE INVENTION

In the method for micro-patterning of a silicon-based elastomer according to an embodiment of the present invention, an initiator is formed at a position of a silicon-based elastomer with high light transmittance and transparency, and a laser beam is moved to induce chain thermal decomposition, thereby performing micro-patterning in a very fast time. It is possible to form a micro pattern having high quality while performing the same.

The laser micro-patterning device according to another example of the present invention is configured to have an intersection point where the first axis laser beam, the second axis laser beam, and the third axis laser beam intersect at one point, and the initiator as the starting point and the intersection point move in three dimensions to form a three-dimensional micro pattern through chain pyrolysis.

The micropatinin chip according to another example of the present invention may provide a microfluidic chip, a cell culture chip, or an organoid having a micro pattern manufactured through chain pyrolysis using a laser beam.

On the other hand, even if it is an effect not explicitly mentioned herein, it is added that the effects described in the following specification expected by the technical features of the present invention and their potential effects are treated as described in the specification of the present invention.

### BRIEF DESCRIPTION OF THE DRAWING

FIG.1 is a photograph showing a surface-processed micro device using a pulse laser according to the prior art.
FIG.2 is a schematic configuration diagram of a micro-patterning device of the present invention capable of forming a micro-pattern by irradiating a laser beam on a silicon-based elastomer.
FIG.3 is a schematic configuration diagram of another example of a micro-patterning device of the present invention capable of forming a micro-pattern by irradiating a laser beam on a silicon-based elastomer.
FIG.4 is a schematic reference diagram of another example of a micro-patterning device of the present invention capable of forming a three-dimensional micro pattern by irradiating a laser beam on a silicon-based elastomer.
FIG.5 is a schematic diagram of a process of forming a micro pattern through chain pyrolysis on a silicon-based elastomer according to the micro-patterning method of the present invention.
FIG.6 is a reference diagram of various examples for forming an initiator on an incident surface of a laser surface on a PDMS slab according to the micro-patterning method of the present invention and forming a micro pattern through chain pyrolysis.
FIG.7 is a reference diagram of various examples for forming an initiator on a surface opposite to an incident surface of a laser beam on a PDMS slab according to the micro-patterning method of the present invention and forming a micro pattern through chain pyrolysis.
FIG.8 and FIG.9 are photographs of PDMS micro pattern chips in which micro patterns are formed by irradiating a laser beam to induce continuous thermal decomposition according to the micro-patterning method of the present invention.
FIG. 10 is a photograph showing a PDMS micro pattern chip and vascular cells cultured in vitro using the micro pattern by inducing continuous thermal decomposition by irradiating a laser beam according to the micro-patterning method of the present invention.
FIG. 11 is a PDMS micro pattern chip formed with a micro pattern by inducing continuous thermal decomposition by irradiating a laser beam according to the micro-patterning method of the present invention by (a) ultra sonication, or (b) removing it by a taping method or a physical external force; It is a picture that shows
FIG. 12 shows the XRD analysis result of the thermal decomposition product removed by the method of FIG.
FIG. 13 shows a result of patterning using laser beams of various wavelengths according to an example of the present invention.
FIG. 14 is a graph showing the correlation between the scanning power of the laser beam and the size of the channel of the micro pattern formed according to the present invention.
FIG. 15 is a graph showing the correlation between the energy density (power density) of a laser beam and the tomographic depth of the channel of the micro pattern formed according to the present invention.
FIG. 16 is a result of measuring the surface shape and turbidity of the micro pattern formed according to the present invention.
FIG.17 is a table showing various embodiments of the micro-patterning chip manufactured according to the present invention.
FIG. 18 shows a blood vessel chip array among micro-patterning chips manufactured according to the present invention.
FIG. 19 shows the results of patterning (a) PDMS or (b) Ecoflex^{®} (ecoflex^{®}, BASF) by irradiating a laser beam according to the micro-patterning method of the present invention to induce continuous thermal decomposition.

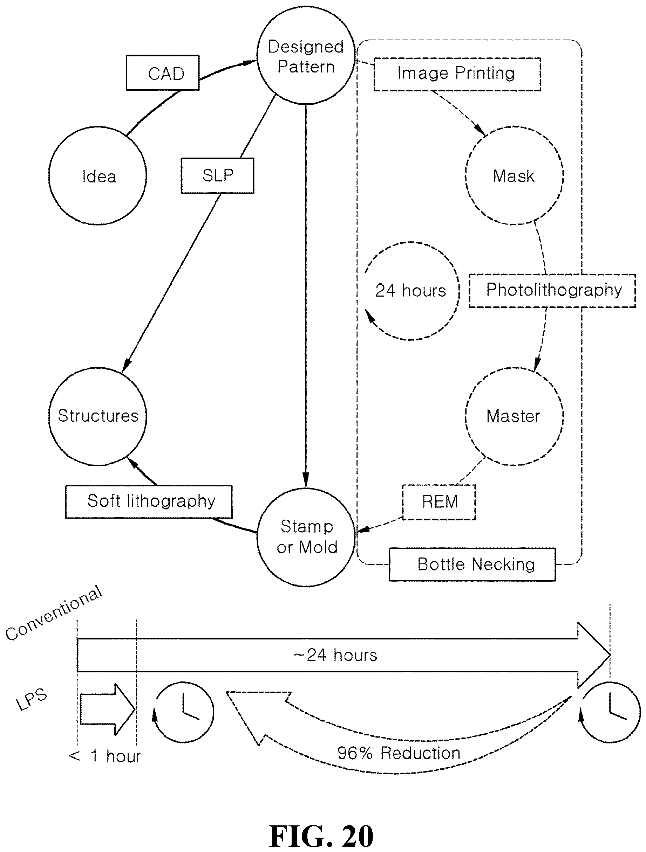

FIG.20 schematically shows a micro-patterning method (LPS) using a laser beam of the present invention compared to a method using conventional lithography.

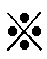 It is revealed that the attached drawings are exemplified as a reference for understanding the technical idea of the present invention, and the scope of the present invention is not limited thereby.

### MODES FOR CARRYING OUT THE INVENTION

Hereinafter, the configuration of the present invention guided by various embodiments of the present invention and effects resulting from the configuration will be described with reference to the drawings. In the description of the present invention, if it is determined that related known functions are obvious to those skilled in the art and may unnecessarily obscure the gist of the present invention, the detailed description thereof will be omitted.

### MICRO-PATTERNING DEVICE

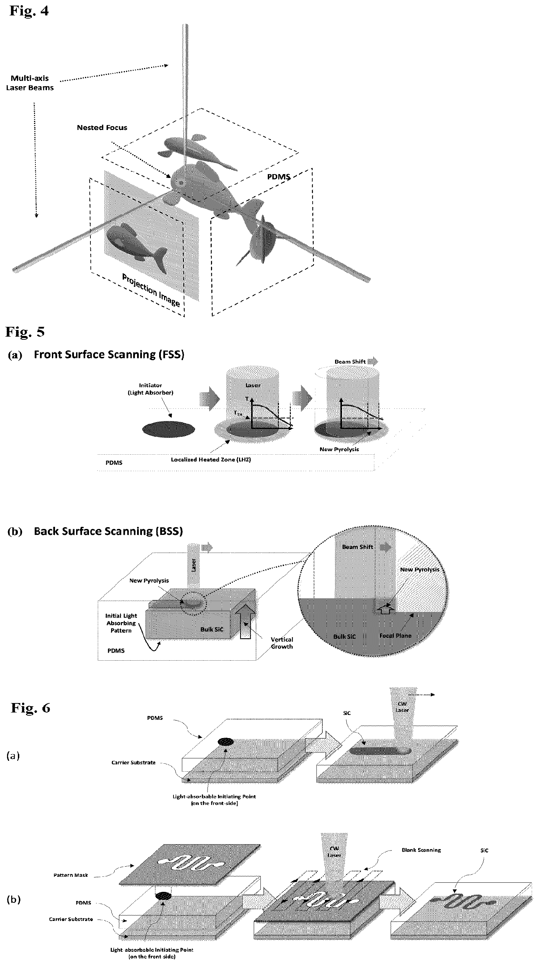

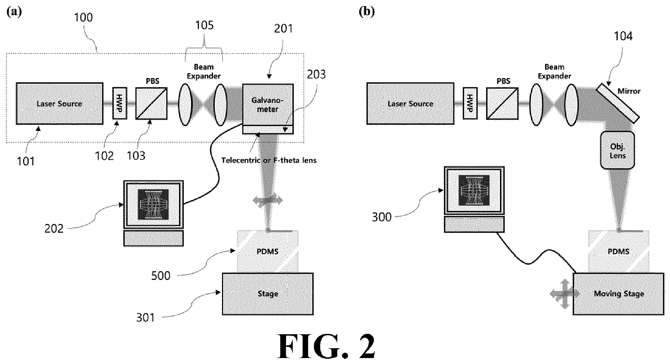

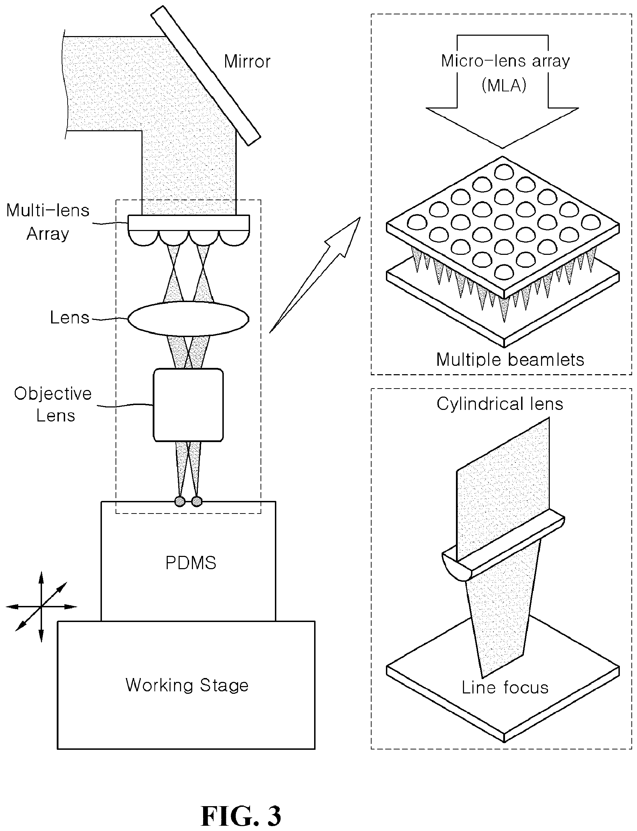

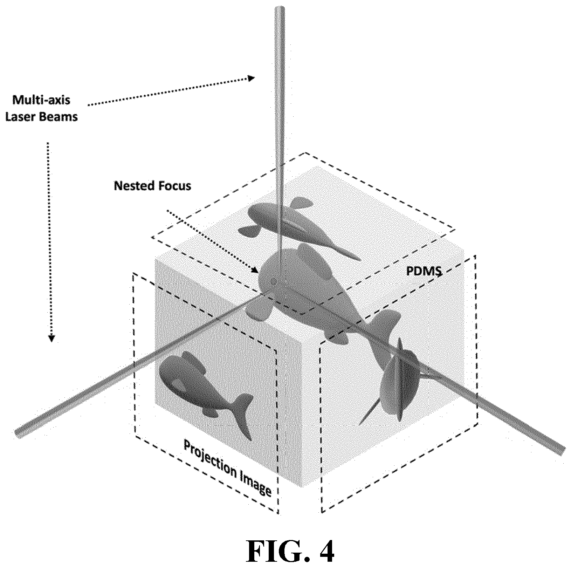

FIG.2 is a schematic configuration diagram of a micro-patterning device of the present invention capable of forming a micro pattern by irradiating a laser beam on a silicon-based elastomer, and FIG.3 is a schematic configuration diagram of another example of a micro-patterning device of , and FIG.4 is a schematic reference diagram of another example of a micro-patterning device of the present invention capable of forming a three-dimensional micro pattern by irradiating a laser beam on a silicon-based elastomer.

First, a micro-patterning device of the present invention will be described with reference to FIG.2 -FIG.4.

First, referring to FIG.2(a), the micro-patterning apparatus of the present invention includes a laser beam generating unit 100, a stage 301, and a control unit. Meanwhile, the laser beam generating unit 100 includes a laser oscillator 101 generating a laser beam, a waveplate 102, a polarized beam splitter (PBS) 103, a mirror unit 104, a beam expander 105 and a laser beam scanner 201 may be provided, but the present invention is not limited thereto. As the laser beam scanner 201, a galvanometer scanner may be used.

The laser oscillator 101 is composed of an optical amplifier and an optical resonator, and outputs a continuous wave laser or a pulsed laser. In the case of a pulse laser, an ultra-short pulse laser may be preferably used.

The wave plate 102 is a module for transmitting a laser beam without attenuation, path deviation, or position change and for adjusting the polarization direction. As the wave plate, a half wave plate (HWP) or a quarter wave plate (QWP) may be used, but the present invention is not limited thereto. The polarization beam splitter 103 is to provide a laser beam incident on an incident surface to first and second output beams through different first and second output surfaces, respectively. The power of the laser can be controlled by the laser source, but it is also possible to control the power of the laser beam using a wave plate and a polarizing beam splitter for stable operation of the source. That is, as the laser oscillated from the laser source passes through the wave plate, the polarization is adjusted at a specific angle, and as the laser passes through the polarization beam splitter, it is divided into two beams. In this case, the intensity ratio of the two output beams can be adjusted according to the polarization angle, and in this way, the power of the output beam to be used can be precisely adjusted.

The mirror unit 104 is a module for expanding the beam incident on the beam expander 105 to be larger than when outputting it in order to change the direction of the laser beam. However, the mirror unit may be omitted when a laser source is directly connected or a fiber laser is used.

The beam expander 105 may be used together when a galvanometer scanner is used, and may be omitted in the case of other methods.

In the present invention, a galvanometer scanner may be used as the laser beam scanner 201. The galvanometer scanner can be used for operations that require rapid scanning, and includes a driving motor that enables the galvanometer scanner to move in the x, y, and z-axis directions. Include in this case, a telecentric lens or an F-theta lens may be used as the lens. However, the present invention is not limited thereto, and various types of lenses such as a plurality of lenses arrayed as shown in FIG.3 or a cylindrical lens may be used.

The laser beam scanner controller 202 controls driving of the laser beam scanner that irradiates the laser beam output from the laser oscillator toward the workpiece to process the workpiece. The laser beam scanner control unit 202 may be interconnected to enable bidirectional communication with the laser beam scanner by wired or wireless communication means, such as a computer, a laptop, a network-connected storage, a mobile device (eg, a tablet device, a smart phone), etc. A general-purpose external terminal including

On the other hand, as shown in FIG.2(b), a laser beam scanner composed of a general objective lens may be used instead of using a galvanometer scanner for more precise precision processing. In this case, it may be more desirable that the stage, not the scanner, be driven (in the x-axis, y-axis, or z-axis). In this case, the stage controller 300 controls the driving device to drive the stage along the x-axis, y-axis, or z-axis.

On the stage 301, a workpiece 500 for forming a micro pattern is placed. In the present invention, a silicone-based elastomer may be used as a workpiece. In particular, a silicone-based elastomer in which an initiator is formed as a workpiece is used.

Referring to FIG.4 , when high-precision 3D scanning is required, the laser beam generator of the micro-patterning apparatus generates a first laser beam generator that generates a first-axis laser beam and a second laser beam that generates a second-axis laser beam and a third laser beam generating unit configured to generate a third axis laser beam. The first to third laser beam generators are configured to have an intersection point where the first axis laser beam, the second axis laser beam, and the third axis intersect at one point. When one laser beam generator is used, it is not impossible to form a 3D-shaped micro pattern (for forming a 3D-shaped structure), but there are many restrictions due to the incident direction of the fixed laser beam. However, as shown in FIG.4, when an intersection is formed using a plurality of laser beam generators, a micro pattern in a 3D shape can be freely formed, and thus it is possible to easily form a 3D structure of various shapes.

Hereinafter, a method for micro-patterning a silicon-based elastomer using the micro-patterning device of the present invention will be described.

### A MICRO-PATTERNING METHOD

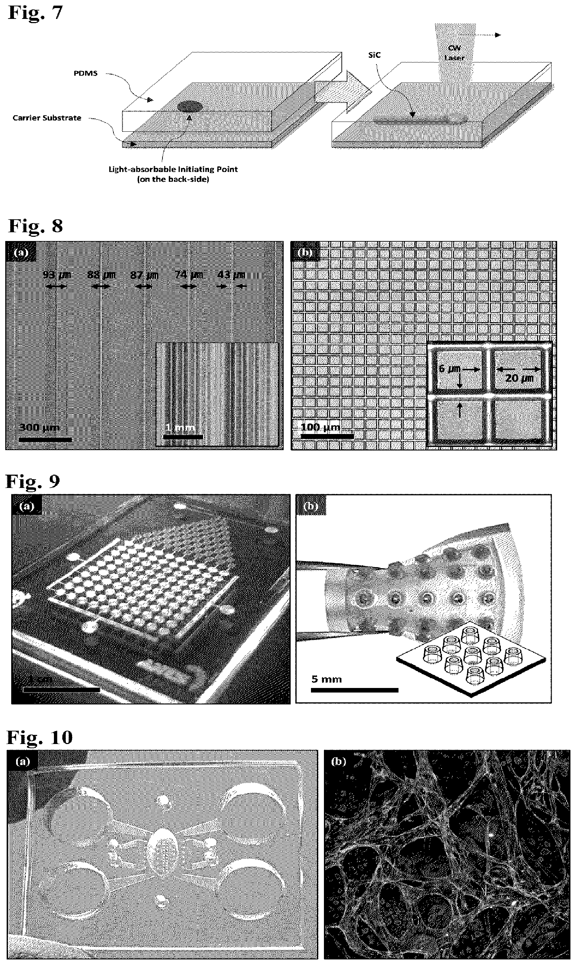

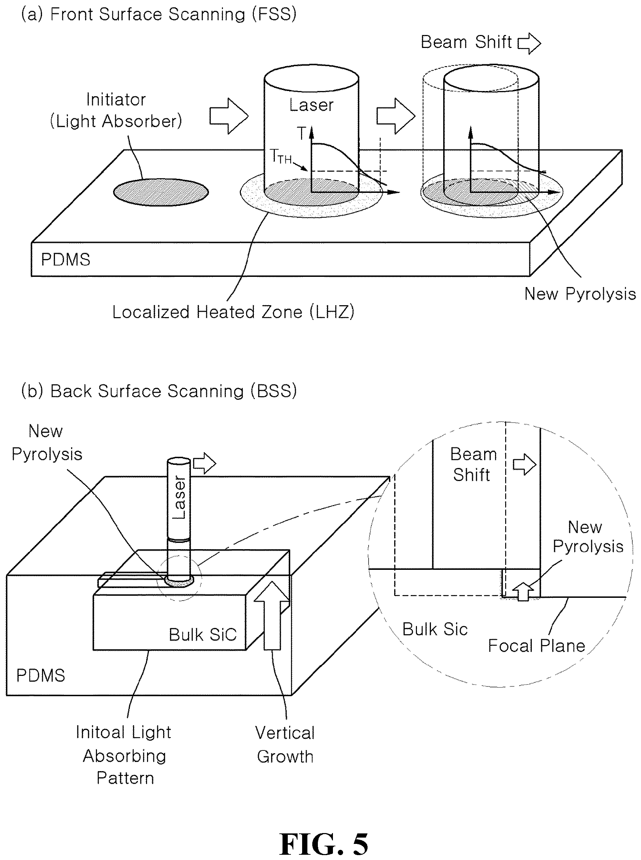

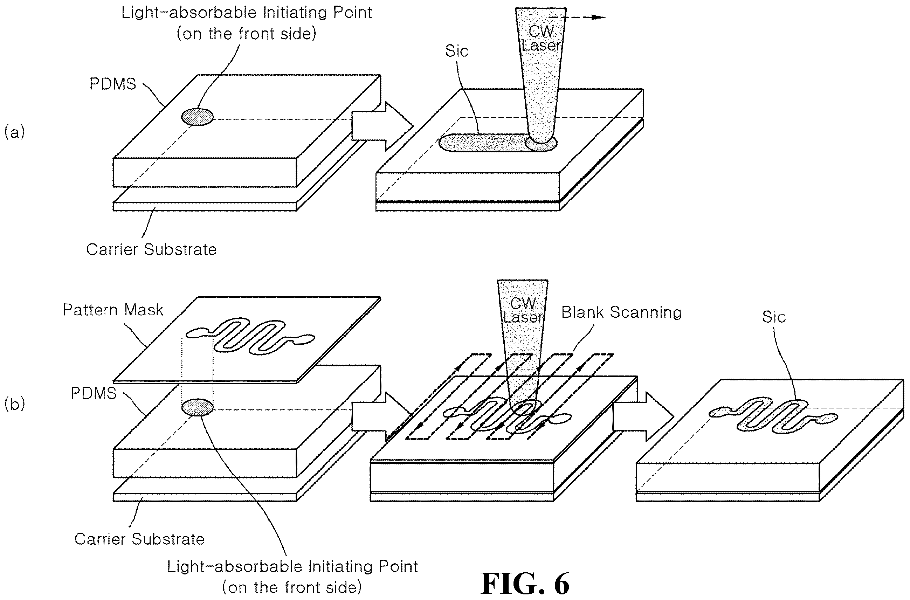

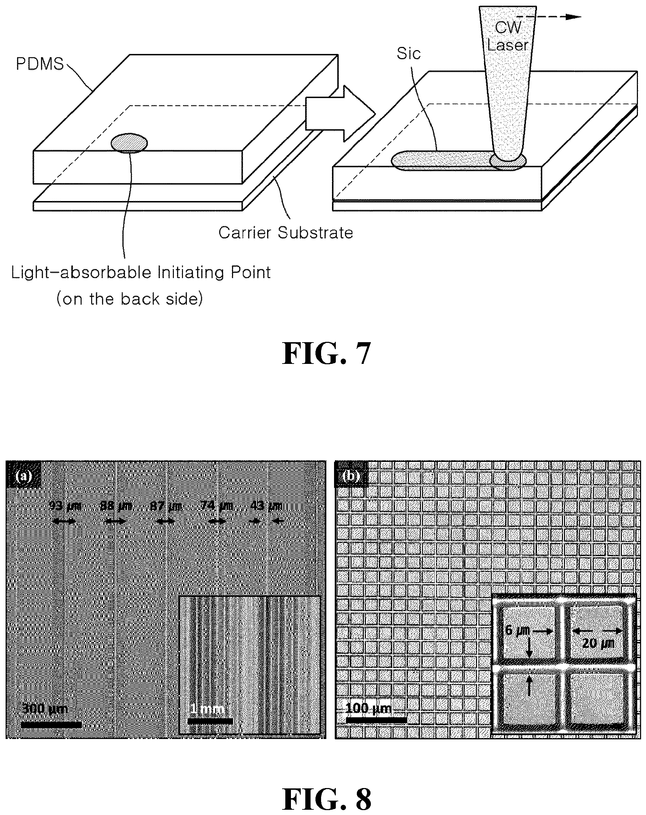

FIG.5 is a schematic diagram of a process in which a micro pattern is formed through chain pyrolysis on a silicon-based elastomer according to the micro-patterning method of the present invention, and FIG. 6 is an initiator on the incident surface of the laser surface on the PDMS slab according to the micro-patterning method of the present invention. It is a reference diagram of various examples for forming a micro pattern and forming a micro pattern through chain pyrolysis, and FIG. 7 is a micro-patterning method of the present invention to form an initiator on the opposite side of the laser beam incident face to the PDMS slab and through chain pyrolysis. It is a reference diagram of various examples for forming a fine pattern.

In the micro-patterning method of the present invention, an initiator is first formed at a position of a silicon-based elastomer. The target of the micro-patterning method of the present invention is a silicon-based elastomer with high light transmittance and transparency, but the silicon-based elastomer with high light transmittance and transparency cannot induce thermal decomposition because the laser beam passes through it. The initiator has light absorption and serves to generate high heat by the laser beam.

Representative examples of silicone-based elastomers include PDMS or ecoflex^{®} (BASF). However, the present invention is not limited thereto, and may be applied to other silicone-based elastomers.

The region in which the initiator is formed is referred to as a first region 1. The initiator may be formed at any position on the silicone-based elastomer, and may be located, inserted, or embedded on the surface of the silicone-based elastomer.

As the initiator, a colored pigment that absorbs light, a dye, an ink, or a solution or solid material containing at least one of these may be used. However, the present invention is not limited thereto, and a material capable of absorbing a laser having a higher light absorption rate than that of the silicon-based elastomer itself may be used.

Next, a laser beam is irradiated to the initiator using a micro-patterning device that irradiates a laser beam to induce a first thermal decomposition in the first region 1. In the silicon-based elastomer of the first region 1 by the first thermal decomposition, SiC or trace amounts of SiOC, SiO2, and nonstoichimetric silca are formed. At this time, a Localized Heated Zone (LHZ) is formed around the first region 1 due to heat transfer, which may be referred to as the second region(2). Although the initiator is not formed in the second region 2, a second thermal decomposition occurs due to heat transfer, so that light absorption is possible.

When the laser beam is moved to the second region 2, light absorption occurs in the second region 2. Heat generated by light absorption is transferred to the periphery of the second region 2 to form a new LHZ around the second region 2. That is, a new second region 2 capable of absorbing light is further formed around the second region 2 capable of absorbing light formed around the first region 1. That is, chain pyrolysis is induced according to the movement of the laser beam. The second pyrolysis is generated by the transfer, so that light absorption is possible.

Therefore, the micro-patterning device of the present invention can be used for micro-patterning of a silicon-based elastomer by using a laser beam like a pen or a brush. For example, the micro-patterning apparatus of the present invention can form a micro-pattern very efficiently and quickly by using a laser beam like one brush drawing using a writing instrument.

In addition, it is also possible to form a fine pattern having a height difference by repeatedly scanning a portion scanned by the laser beam or varying the scanning time of a specific location. More specifically, it is possible to implement a gray-scale lithography technique that forms structures of various aspect-ratios by adjusting the laser intensity or scanning speed for each location, or by using repeated scanning to form a structure of various aspect-ratios even in a single patterning process.

FIG.6 schematically illustrates a micro-patterning method when an initiator is formed on an incident surface of a silicon-based elastomer. The silicon-based elastomer has a first surface that is an incident surface to which a laser beam is irradiated, and a second surface that faces the first surface. FIG.6(a) and FIG.6(b) are examples in which the initiator is formed on the first surface.

Referring to FIG.6(a), a fine pattern is formed by irradiating a laser beam to the initiator to induce a first thermal decomposition followed by a second thermal decomposition. A fine pattern is formed according to the path of the laser beam.

In contrast, FIG.6(b) uses a mask in which holes corresponding to the fine patterns are formed. An initiator is formed on the first surface of the silicone-based elastomer, and a mask is placed at the same time. Then, the laser beam is subjected to blank scanning. Rather than moving the laser beam according to the fine pattern, the laser beam scans the entire surface from left to right. Since there is a hole corresponding to the micro pattern in the mask, the micro pattern is formed starting from the position where the hole initiator is during blank scanning.

FIG.7 schematically illustrates a micro-patterning method when an initiator is formed on a surface opposite to an incident surface of a silicon-based elastomer. That is, it is an example in which the initiator is formed on the second surface based on FIG.6(a) and FIG.6(b).

As a FIG.7, when the initiator is formed on the surface opposite to the incident surface of the silicon-based elastomer, there is an advantage that it is preferable to form a 3D structure.

When the initiator is formed on the incident surface of the silicon-based elastomer, after the thermal decomposition product is formed by thermal decomposition, it is difficult to induce thermal decomposition in the depth direction because the laser is difficult to penetrate.

However, when the initiator is formed on the surface opposite to the incident surface of the silicon-based elastomer, there is no problem in that the laser beam still passes through the silicon-based elastomer even when SiC is formed by thermal decomposition. For example, it is also possible to form a conical micro pattern by repeatedly drawing a circle several times while gradually narrowing the area over it after forming a large circular pyrolysis region by irradiating a laser beam to the initiator on the opposite surface.

Referring to FIG.7, a micro pattern is formed by irradiating a laser beam to the initiator to induce the first thermal decomposition and the resulting second thermal decomposition. A fine pattern is formed according to the path of the laser beam. The difference from FIG.6(a) is that the laser beam passes through the silicon-based elastomer and pyrolyzes the opposite surface on the path of the laser beam.

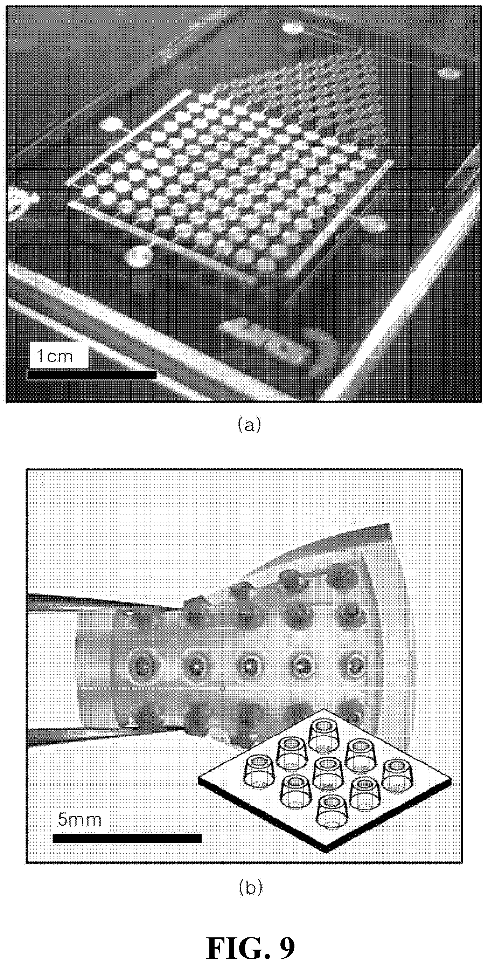

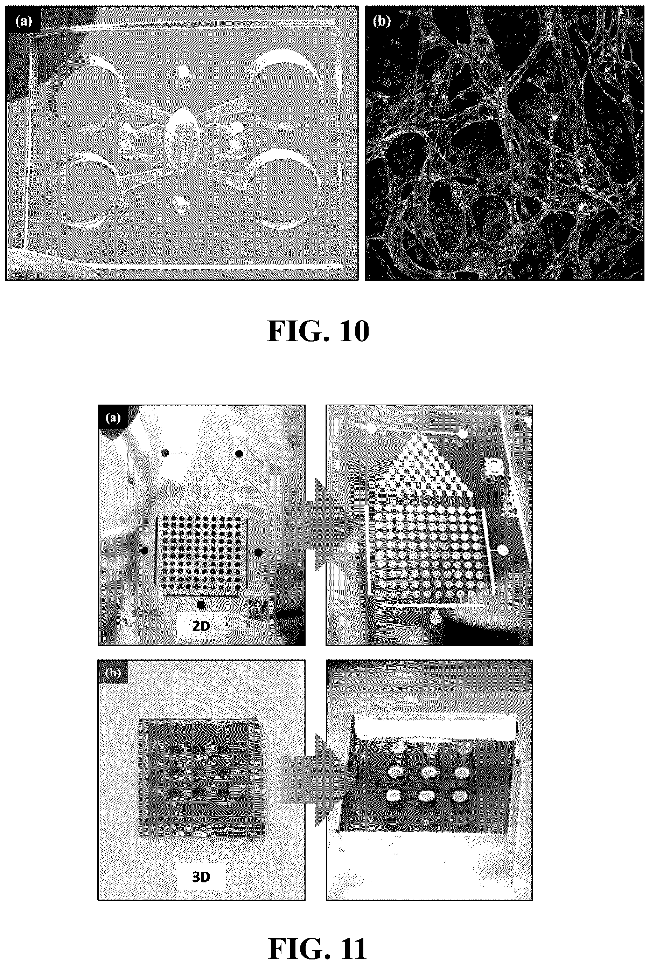

FIG.8 and FIG.9 are enlarged photos of a micro-patterning chip manufactured according to the micro-patterning method of the present invention in various embodiments of the present invention, and FIG. 10 is continuous thermal decomposition by irradiating a laser beam according to the micro-patterning method of the present invention. This is a photograph showing a PDMS micro pattern chip and vascular cells cultured in vitro using the micro pattern by inducing. In particular, FIG. 10 shows that blood vessels are stably formed by culturing blood vessel cells in the micro-patterning chip manufactured according to the method of the present invention.

These various embodiments of the present invention are only for illustrating the present invention, and other micro-patterning chips that can be manufactured by those skilled in the art to which the present invention pertains after understanding the content of the present invention will be included in the present invention. On the other hand, the unique characteristics of the micro-patterning chip manufactured according to the micro-patterning method of the present invention will be described later through examples.

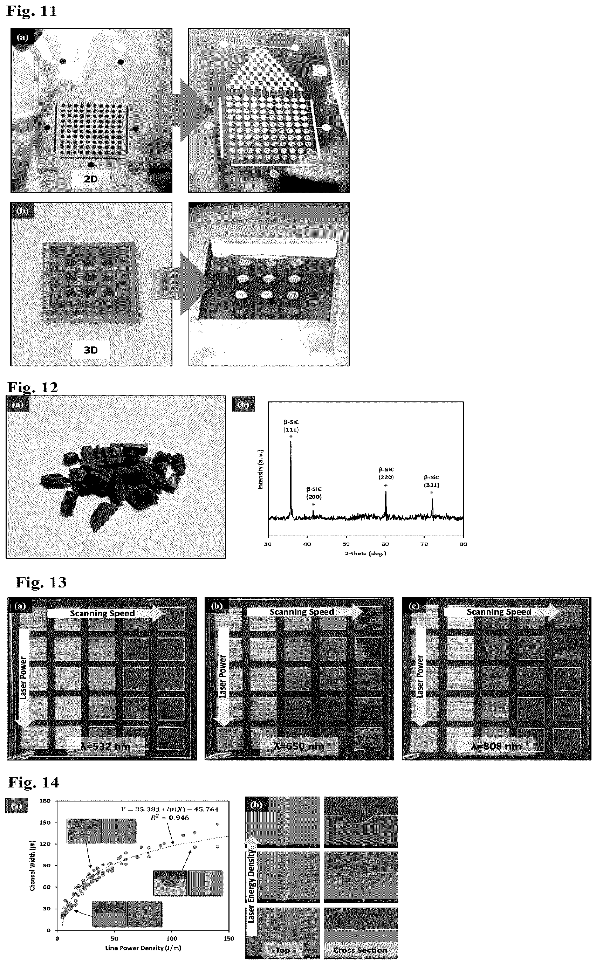

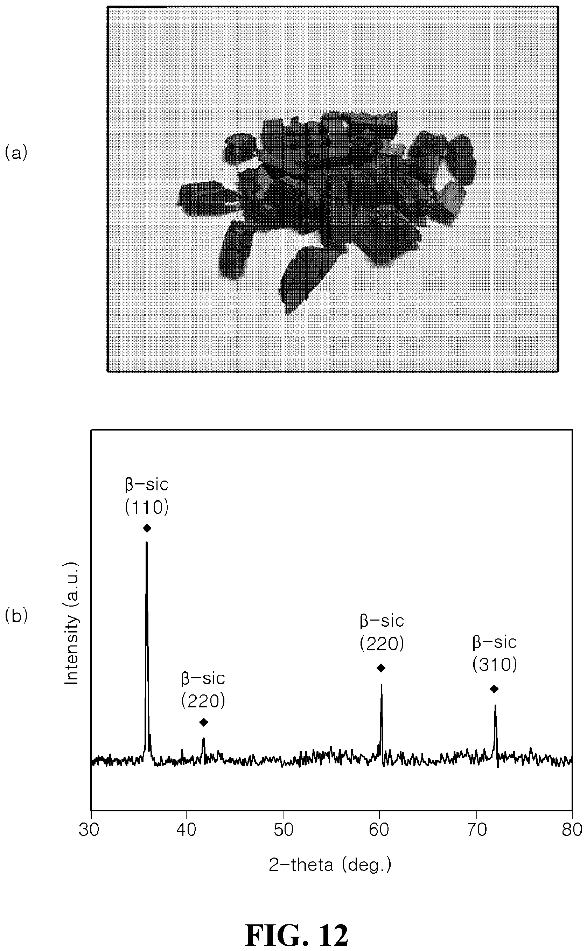

FIG. 11 is a PDMS micro-pattern chip, which is irradiated with a laser beam to induce continuous thermal decomposition according to the micro-patterning method of the present invention to form a micro pattern, (a) ultra sonication, or (b) a taping method or a physical external force. It is a photograph showing, and FIG. 12 shows the XRD analysis result of the pyrolysis product removed by the method of FIG. 11.

When a micro pattern is formed by irradiating a laser beam, a thermal decomposition product is generated in the area where the micro pattern is formed. As a result of XRD analysis of the thermal decomposition products, it was confirmed that most of them were SiC or SiOC, and a small amount of SiO2 or amorphous silica was formed.

Such thermal decomposition products are very easily separated from the silicone-based elastomer. The thermal decomposition product is desorbed just by slightly bending the silicone-based elastomer.

However, in order to more cleanly remove the thermal decomposition product, it is also possible to remove the thermal decomposition product from the silicone-based elastomer by using ultrasonic waves in a state of being immersed in ethanol, or to remove the thermal decomposition product by using a tape or external force.

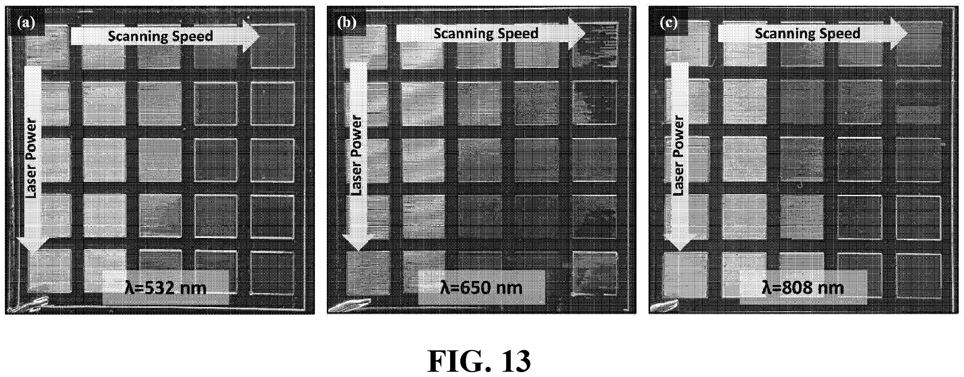

FIG. 13 shows a result of patterning using laser beams of various wavelengths according to an example of the present invention. In FIG. 13, a laser beam having a wavelength of 532 nm, 650 nm, and 808 nm, respectively, was used in an embodiment of the present invention, and it was confirmed that good fine patterns were generated regardless of the wavelength. Preferably, the wavelength of the laser beam may be 200 nm to 1,000 nm.

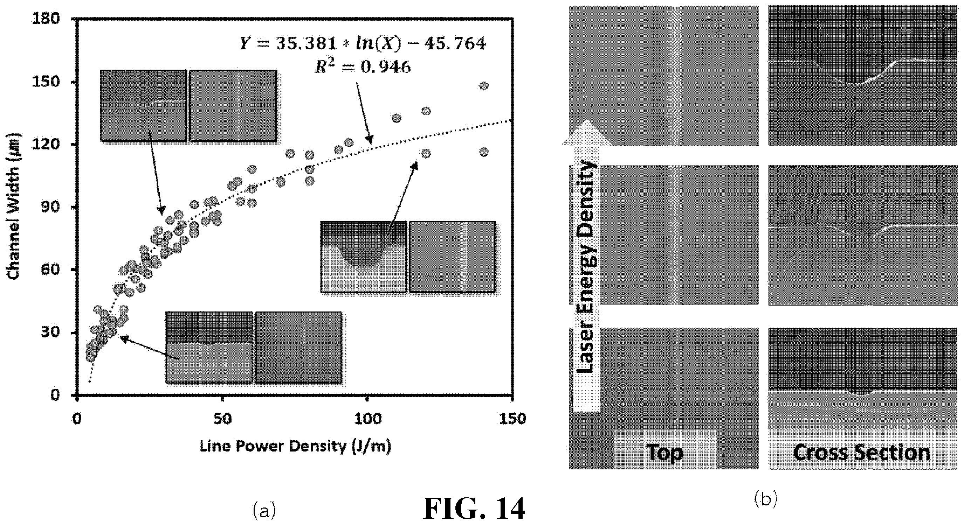

FIG. 14 is a graph showing the correlation between the scanning power of a laser beam and the size of a channel of a micro pattern formed according to the present invention. In FIG. 14, as the scanning power (J/m) of the laser beam increases, the depth and width of the microchannel increase in proportion to it, but it can be seen that the increase rate of the width is greater.

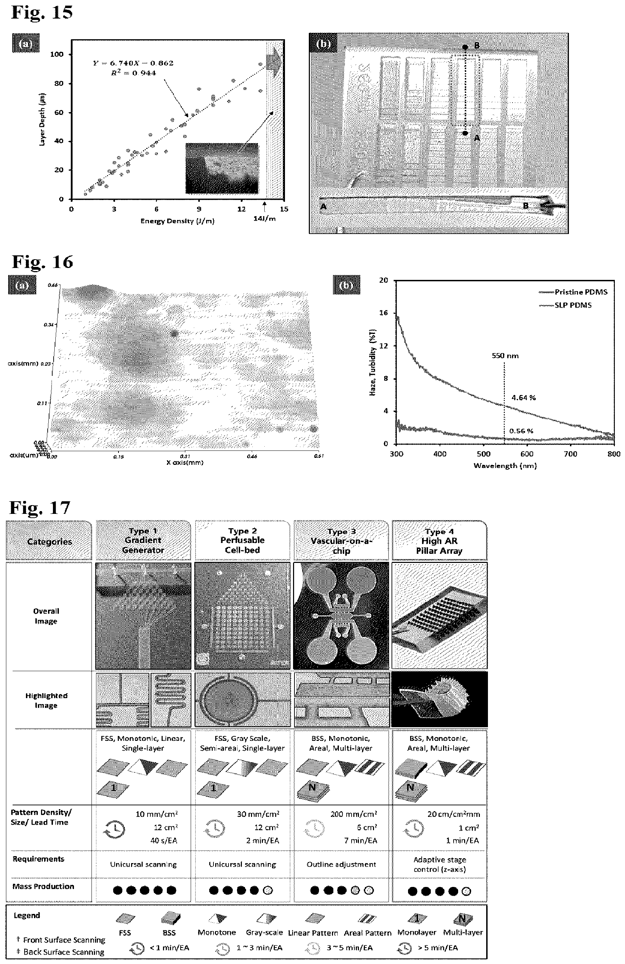

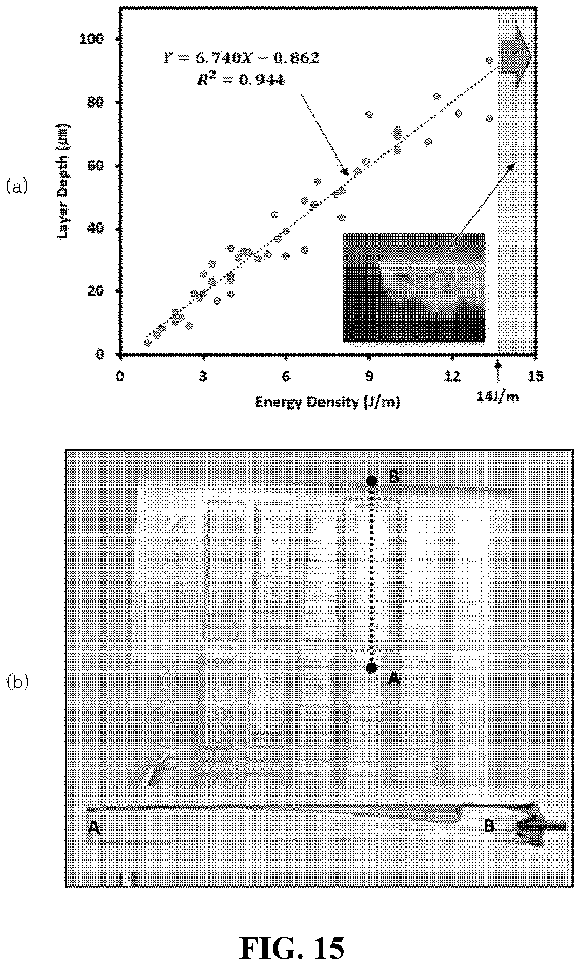

FIG. 15 is a graph showing the correlation between the energy density (power density) of a laser beam and the tomographic depth of the channel of the micro pattern formed according to the present invention. Referring to FIG. 15, it was found that the tomographic depth of the channel formed by the laser beam increases in proportion to the energy density (J/m) of the laser beam.

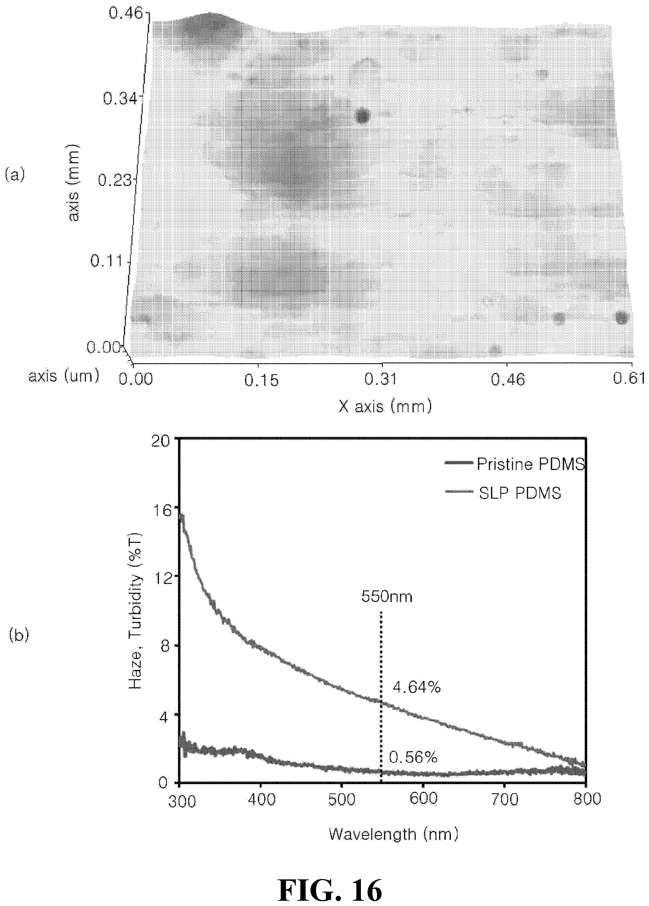

FIG. 16 is a result of measuring the surface shape and turbidity of the micro pattern formed according to the present invention. Referring to FIG. 16(a), it can be seen that the micro-patterning method of the present invention inevitably forms small grooves on the surface of the micro-pattern. Such grooves make the surface of the micro pattern to have a slightly opaque surface. As a result, as can be seen from FIG. 16(b), in the haze characteristic, the micro pattern has a turbidity of 4%T or more, compared to a typical PDMS having a turbidity of about 0.5%T. The position where the micro pattern is formed has a turbidity difference of about 8 times or more compared to the general PDMS.

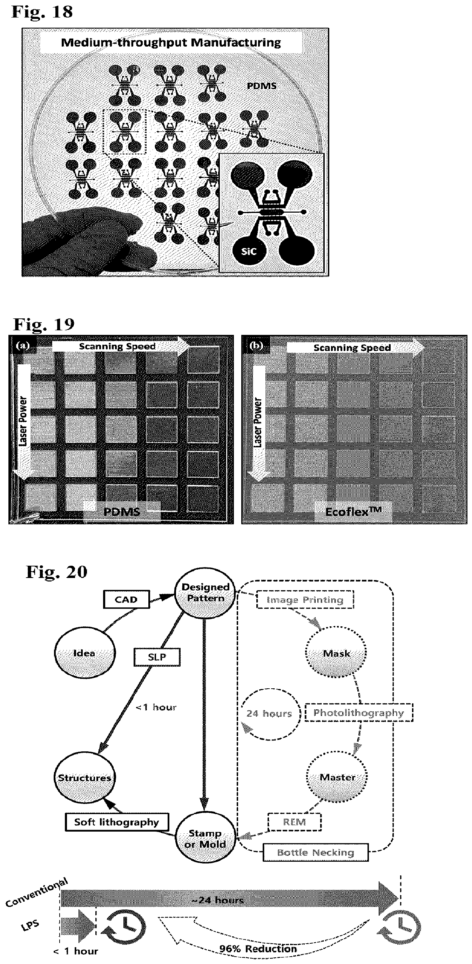

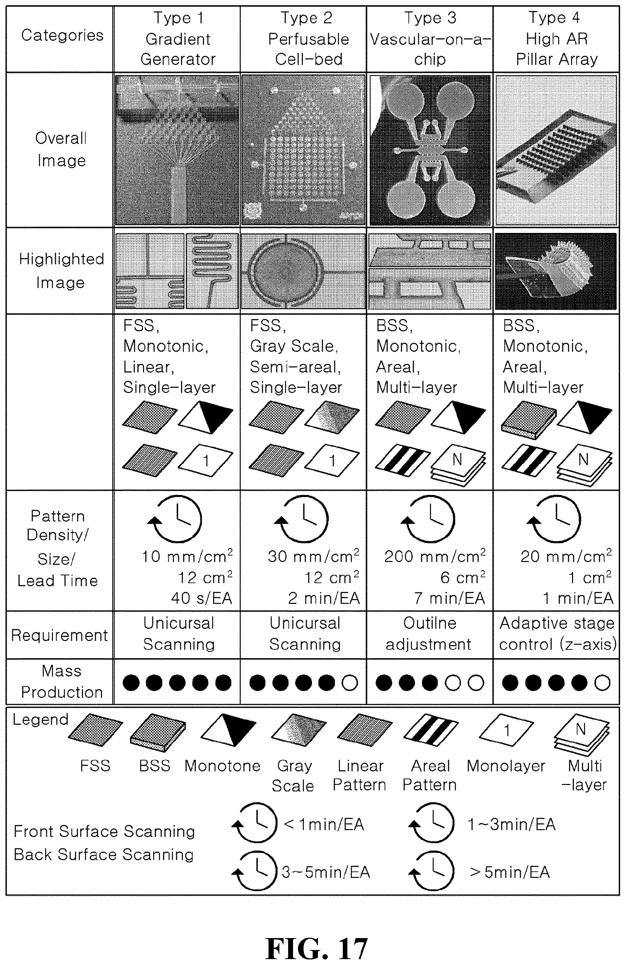

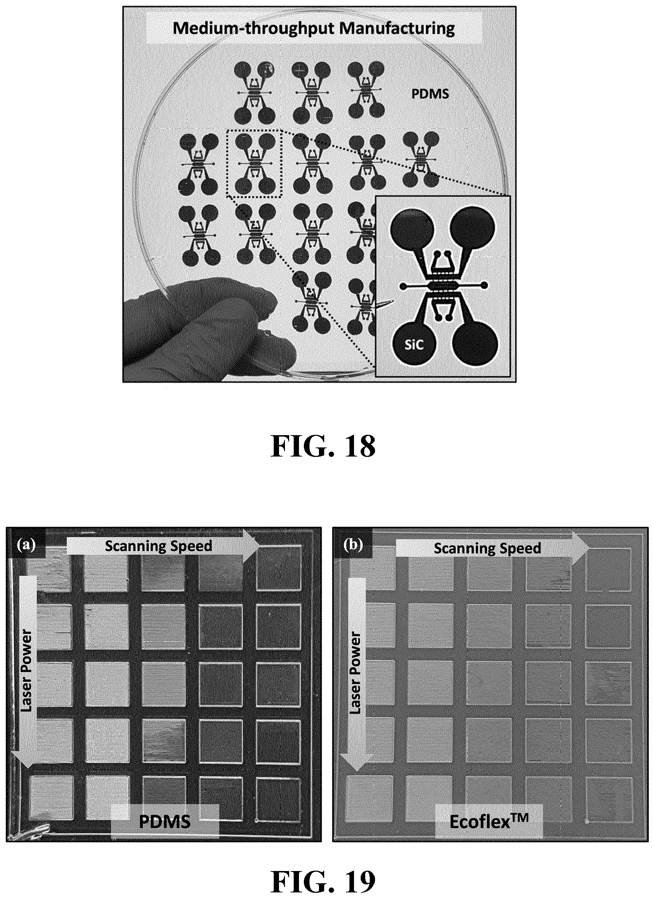

FIG.17 is a table showing various embodiments of the micro-patterning chip manufactured according to the present invention, and FIG. 18 shows a blood vessel chip array among the micro-patterning chips manufactured according to the present invention.

The microfluidic chip, cell culture chip, etc. including FIG. 17 and FIG. 18 may be manufactured by the micro-patterning method of the present invention described above.

FIG. 19 shows the results of patterning (a) PDMS or (b) Ecoflex^{®} (ecoflex^{®}, BASF) by irradiating a laser beam according to the micro-patterning method of the present invention to induce continuous thermal decomposition. PDMS and Ecoflex are representative silicone-based elastomers. Referring to FIG.19, in both cases, it was confirmed that an excellent level of fine pattern was formed by the micro-patterning method of the present invention.

### Example

### <Example 1: Preparation of PDMS slab>

A PDMS slab was prepared by mixing a resin ((Dow Corning)) and a curing agent (Sylgard184, Dow Corning) in a ratio of 10:1. Next, degassing and curing processes were sequentially performed at 60 °C for 2 hours or more using a vacuum bell-jar and curing oven (OF-12G, JEIO TECH).

### <Example 2: Configuration of micro-patterning device>

In this example, two types of computer-controlled laser beam scanning systems were used. For fast patterning, a galvanometer (hurrySCAN II, Scanlab) with a telecentric lens (f=103 mm) was used, and for fine patterning of 10 µm or less, a computer-controlled 2-axis stage (ANT130-060-) XY-25DU-XY-CMS-MP-PLUS, Aerotech) and a high magnification objective (M Plan Apo 50X, Mitutoyo) were used. For both above, a continuous wave laser (532 nm, Sprout-G-5W, Lighthouse Photonics) was used as the main laser source, compared to 650 nm and 808 nm.

### <Example 3: Fabrication of micro-patterning chip>

The cured PDMS slab prepared in Example 1 was cleaned by a taping (Scotch Magic Tape, 3M) method to remove foreign substances such as dust attached to the surface. The surface-treated PDMS slab was placed on a glass used as a transport substrate. Computer-controlled laser scanning having appropriate scanning parameters such as power or scanning speed was used as FSS (front surface scanning, see FIG. 6(a)) or BSS (back surface scanning, see FIG.7) method. For each method, the laser focus was precisely controlled to enable high-quality machining. For FSS, the focal plane was placed on the beam incident surface of the PDMS slab. In the case of BSS, the laser focal plane was adjusted to the opposite side of the incident surface of the PDMS slab prior to the first scanning, and after that, the thickness of the pyrolysis product newly generated by the scanning was compensated for each scanning. SiC was easily removed by taping or ultra-sonication. The PDMS structure was combined with a slide glass by a standard plasma-bonding method or used as a mold to fabricate a microfluidic chip.

The micro-pattern of the microfluidic chip of Example 3 was able to be formed very quickly, and it was confirmed that the quality of the formed micro-pattern was equal to or higher than that using photolithography.

Therefore, when the micro-patterning method of the present invention is used, it is possible to easily pattern a light-transmitting workpiece, which was impossible or inefficient to process using a conventional laser beam, by a method such as one-stroke drawing.

The protection scope of the present invention is not limited to the description and expression of the embodiments explicitly described above. In addition, it is added once again that the protection scope of the present invention cannot be limited due to obvious changes or substitutions in the technical field to which the present invention pertains.

## Claims

1. A method for micro-patterning of a silicone-based elastomer, comprising:
forming an initiator having a first region at one position of the silicone-based elastomer,
wherein a laser beam from a micro-patterning device irradiating a laser beam is irradiated to the initiator to induce a first thermal decomposition in the first region, but a second thermal decomposition occurs outside the first region by conduction of heat generated in the first thermal decomposition. to form a second region capable of absorbing light,
wherein the laser beam is irradiated while moving to a second region to form a micropattern.

2. A method for micro-patterning of a silicone-based elastomer according to claim 1,
wherein the silicon-based elastomer has a first surface that is an incident surface to which a laser beam is irradiated, and a second surface that faces the first surface,
wherein the initiator is formed on the first surface of the silicon-based elastomer to form a 2D micropattern while the laser beam moves in the 2D region.

3. A method for micro-patterning of a silicone-based elastomer according to claim 1,
wherein the silicon-based elastomer has a first surface that is an incident surface to which a laser beam is irradiated, and a second surface that faces the first surface,
wherein the initiator is formed on the second surface of the silicon-based elastomer to form a 3D micropattern while a laser beam moves in a 3D region.

4. A method for micro-patterning of a silicone-based elastomer according to claim 1,
wherein the micro-patterning device generates a first-axis laser beam, a second-axis laser beam, and a third-axis laser beam,
wherein the first-axis laser beam, the second-axis laser beam, and the third-axis laser beam intersect at one point, forming a 3D micropattern while moving in a 3D region using the initiator at one position of the silicon-based elastomer as a starting point.

5. A method for micro-patterning of a silicone-based elastomer according to claim 1,
wherein the initiator is a solution or a solid material having a higher light absorption than the silicon-based elastomer.

6. A method for micro-patterning of a silicone-based elastomer according to claim 1,
wherein the initiator is positioned on the surface of the silicone-based elastomer or inserted or embedded into the silicone-based elastomer.

7. A method for micro-patterning of a silicone-based elastomer according to claim 1,
removing a thermal decomposition product after forming the micropattern.

8. A method for micro-patterning of a silicone-based elastomer according to claim 7,
wherein the pyrolysis product is SiC, SiOC, SiO2, or amorphous silica.

9. A method for micro-patterning of a silicone-based elastomer according to claim 1,
wherein the silicon-based elastomer is PDMS (Poly (methylmethacrylate)).

10. A method for micro-patterning of a silicone-based elastomer according to claim 1,
wherein the laser beam is a continuous wave laser beam or a pulsed laser beam.

11. A method for micro-patterning of a silicone-based elastomer according to claim 1,
wherein the power density of the laser beam is less than the power density for melting the silicon-based elastomer.

12. A micropatterning chip manufactured by the method of micropatterning the silicon-based elastomer according to any one of claims 1 to 11,
wherein the micropattern formed has a turbidity of 4%T or more to light at 550 nm.

13. A micro-patterning device inducing chain thermal decomposition in a silicon-based elastomer to form a fine pattern, which comprises a laser beam generating unit, a stage, and a laser beam generating unit or a control unit for controlling the stage,
wherein the laser beam generator includes a laser oscillator; beam expander; and a laser beam scanner;
and it is mounted on the stage and includes a silicon-based elastomer having at least one initiator for absorbing light as a workpiece mounted on the stage and on which a fine pattern is formed by the laser beam,
wherein the laser beam scanner or the stage or both are driven in a 2D area or a 3D area by a driving device.

14. A micro-patterning device according to claim 13,
wherein the laser beam generator includes a first laser beam generator that generates a first axis laser beam, a second laser beam generator that generates a second axis laser beam, and a third laser beam generator that generates a third axis laser beam, and the first to third axis laser beam from each generator are configured to make an intersection at one point.

15. A micro-patterning device according to claim 13,
wherein the laser beam scanner is a galvanometer scanner.

16. A micro-patterning device according to claim 13,
wherein the wavelength of the laser beam generated by the laser beam generator is 200 nm to 1,000 nm.

17. A micro-patterning device according to claim 13,
wherein the energy density of the laser beam generated by the laser beam generator is 10 to 100 J/m.
